Europäisches Patentamt

European Patent Office

Office européen des brevets

(11) Publication number: **0 482 261 B1**

# EUROPEAN PATENT SPECIFICATION

(45) Date of publication of patent specification: **04.01.95**  (51) Int. Cl.6: **A61M 16/00, A61M 16/12**

(21) Application number: **90311793.5**

(22) Date of filing: **26.10.90**

(54) **Ventilator control system.**

(43) Date of publication of application:
**29.04.92 Bulletin 92/18**

(45) Publication of the grant of the patent:
**04.01.95 Bulletin 95/01**

(84) Designated Contracting States:
**AT BE CH DE DK ES FR GB GR IT LI LU NL SE**

(56) References cited:
**EP-A- 0 117 699**
**EP-A- 0 256 171**
**GB-A- 2 079 984**

(73) Proprietor: **PURITAN-BENNETT CORPORATION**
**2310 Camino Vida Roble**
**Carlsbad,**
**California 92009 (US)**

(72) Inventor: **Forare, Lester**
**4632 Hugins Way,**
**San Diego, California 92122 (US)**
Inventor: **Gagne, Roger**
**2773 Levants Street**
**Carlsbad, California 92009 (US)**
Inventor: **Fennema, Paul**
**1619 Acacia Lane**
**Fallbrook, California 92028 (US)**

(74) Representative: **Baverstock, Michael George**
**Douglas et al**
**BOULT, WADE & TENNANT**
**27 Furnival Street**
**London, EC4A 1PO (GB)**

## Description

This invention relates generally to breathing ventilators, and more particularly to a pnuematically driven, electronically controlled ventilator system for mixing gas proportions and delivering breathing gas for either controlled or spontaneous breaths.

Conventional breathing ventilators generally provide a positive over pressure of breathing gas to a patient, and may provide respiratory gas under pressure in a predetermined manner responsive to pressure variations in the breathing pattern of the patient, or may operate to supply a predetermined volume of respiratory gas to a patient in a controlled manner for each breath. The breathing gas is generally provided with an elevated concentration of oxygen. An oxygen concentrator having a reservoir for receiving the oxygenated product gas, including a sensor for monitoring withdrawal of the product gas from the reservoir, and a microprocessor for determining the minimum time for charging of the gas to provide a product gas with a selected oxygen concentration at the sensed rate of withdrawal is described in U. S. Patent No. 4,561,287 (Rowland). A plurality of canisters with molecular sieve beds for absorbing nitrogen provide the oxygenated gas to the reservoir, and a valve mechanism is provided for directing air from a compressor to alternate sieve beds. Another system for mixing the two gases in predetermined proportions involves the introduction of two gases via separate inlets into a pressure vessel receiving the first gas up to a first pressure, and then receiving the second gas up to a second pressure, to provide the proper proportioning of the mixed gases. This system is described in U.S. Patent Nos. 4,022,234 and 4,023,587 (Dobritz). The mixed gases are withdrawn until the initial pressure within the receiving vessel is reached, at which time the withdrawal of gas is interrupted, and the mixing cycle is again commenced. The feedback control of the rate of flow and pressure of breathing gas to a patient by an inspiration servounit is also described in U.S. Patent No. 3,741,208 (Jonsson, et al.)

It would be desirable to provide a ventilator system which can mix the constituent gases in a respiratory gas in a manner responsive to pressure within the respiratory gas contained in the receiving vessel, and responsive to the various modes of breathing for which the respiratory gas is being provided.

The present invention provides a system and a method for delivery of respiratory gas to a patient from a containment vessel, which includes measurement of the quantity of gas within the containment vessel, control of the flow of respiratory gas into the containment vessel responsive to pressure within the containment vessel, and control of the outflow of respiratory gas responsive to changes in pressure or volume in the containment vessel.

According to the present invention there is provided a ventilator system for the delivery of respiratory gas to a patient comprising: a fixed wall containment vessel; first and second inlet ports connected to said vessel with each said inlet port being in fluid communication with corresponding first and second separate sources of first and second respective component gases to be combined in a desired ratio to form said respiratory gas, with said first and second inlet ports having associated corresponding first and second inlet valves for individually controlling the flow of each said component gas through said inlet ports to said vessel; outlet means to connect said vessel in fluid communication with the patient; sensor means to sense the pressure of said respiratory gas in the containment vessel; inlet flow control means to sequentially control the flow of said first component gas into said containment vessel through said first inlet port while said second inlet port is closed, and to control the flow of said second component gas through said second inlet port while said first inlet port is closed, in said desired ratio responsive to the pressure of gas sensed in the containment vessel, including means for determining a desired pressure change value for each said component gas in said containment vessel corresponding to the number of moles of each said component gas required to fill said vessel with said desired ratio of said component gases, by determining an estimated number of molecules in said vessel from sensed pressure in the vessel and a prior first component gas concentration, and subtracting the estimated number of molecules from a desired number of molecules in said vessel, and means for determining an actual ratio of said component gases in said respiratory gas in said vessel by determining the number of moles of each of said component gases in said vessel based upon sensed changes in pressure of each of said component gases introduced into said containment vessel; and outlet flow control means including microprocessor or electronic means electrically connected to said sensor means for controlling the outflow of said respiratory gas through the outlet means characterized by:

said sensor means including means for electronically differentiating pressure sensed in said containment vessel over a period of time to generate a differentiated pressure signal representing an outflow rate of change of pressure in said containment vessel over a period of time, and said outlet flow control means controlling said outflow of respiratory gas responsive to such differentiated pressure signal based upon the rate of change in the pressure of gas sensed in the containment vessel over a period of time.

The present invention also provides a method for controlling the delivering of respiratory gas to a patient from a ventilator system comprising a fixed wall containment vessel having first and second inlet ports, each of said inlet ports connecting the vessel in fluid communication with first and second separate sources of respective first and second component gases to be combined in said vessel in a desired ratio to form said respiratory gas, said respiratory gas in said vessel having an initial known ratio of said component gases; outlet means to connect the vessel in fluid communication with the patient; sensor means to sense the pressure of said respiratory gas in the containment vessel; means to sequentially control the flow of said component gases through said inlet ports; and output flow control means to control the flow of said respiratory gas through said outlet means, said method comprising the steps of: measuring the pressure of gas within said vessel following inhalation by the patient; determining pressure increase values for said first and second component gases in said vessel corresponding to the number of molecules of each said component gas required to fill said vessel with said desired ratio of said component gases to a desired fill pressure by determining an estimated number of molecules in said vessel from sensed pressure in the vessel and a prior first component gas concentration, and subtracting the estimated number of molecules from a desired number of molecules in said vessel; sequentially filling said vessel with said component gases to said corresponding pressure increase values while measuring pressure in said containment vessel, by introducing said first component gas into said vessel through said first inlet port while said second inlet port is closed, and said second component gas through said second inlet port while said first inlet is closed according to said pressure increase values until said fill pressure of gas in said vessel is reached; measuring an actual pressure increase in said containment vessel for each said component gas introduced into said vessel; determining the ratio of said component gases in said containment vessel by determining the number of moles of each of said component gases in said vessel based upon said initial known ratio of component gases in said vessel, said total pressure and said measured actual pressure changes for each said component gas introduced into said vessel; withdrawing said gas from said vessel through said outlet means; measuring an outflow rate of change in the pressure of gas in the vessel over time; and controlling said withdrawing of gas from said vessel, characterized by:

generating a differentiated pressure signal representing the outflow rate of change of pressure in said containment vessel over a period of time, and said controlling of withdrawing of gas being responsive to said differentiated pressure signal based upon the rate of change in the pressure of gas sensed in said containment vessel over a period of time.

In a preferred embodiment of the system of the invention, means are also included for determining the rate of outflow of gas from the vessel based upon the pressure or volume sensed within the vessel, means for comparing the outflow rate with a predetermined value, and means for adjusting the outflow based upon this comparison. The inlet flow control also preferably includes means for comparing the pressure or volume within the vessel with a predetermined value, and a valve mechanism for filling the vessel responsive to this comparison. In another preferred embodiment of the invention, means are included for determining a predicted pressure or volume in the containment vessel based upon a predicted rate of change in the vessel, with the valve mechanism for filling the vessel being responsive to a predicted pressure or volume. The containment vessel also preferably contains a means for reducing temperature fluctuations in the respiratory gas contained within the vessel.

In the method of the invention, the control of withdrawal of gas from the containment vessel preferably includes comparing a measure of the change in the quantity of gas in the vessel, such as the pressure or volume change, with a corresponding predetermined value and adjusting the outflow in the outlet to reduce the difference between the measured change and the predetermined value. The method preferably includes comparing the measured quantity of gas in the containment vessel with a predetermined threshold value, and introducing gas into the vessel when the quantity of gas falls below the threshold value. Another preferred embodiment of the method involves determining a predicted quantity of gas in the vessel based upon a predicted rate of change in the vessel, comparing the predicted value with a predetermined value, and filling the vessel with gas when the predicted value falls below the predetermined threshold value. The respiratory gas is also preferably composed of a plurality of component gases, and the method preferably includes determining the estimated number of molecules or quantity of gas within the vessel based upon the gas pressure or volume in the vessel, determining the number of moles of each component gas required to fill the vessel to a predetermined fill level with a particular ratio of component gases; for each component gas, determining a pressure change value and pressure increase value in the vessel corresponding to the number of moles required to fill the vessel with the predetermined ratio of component gases; and filling the vessel with each component gas up to the pressure increase value.

Two basic breath types are supported by the ventilator and method of the invention. A mandatory breath pattern is supported, in which the physical characteristics of the breath supplied to the patient are completely specified by operator selected settings. The physical characteristics selected by the operator may include a wave form, tidal volume, peak inspiratory flow, composition, such as the oxygen concentration, and the cycle interval. A mandatory breath pattern can be initiated by an independent operator, or even by the patient.

A spontaneous breath pattern is also supported by the invention. A spontaneous breath is one in which the flow of inspiratory gas and tidal volume are determined solely by the patient effort. The composition of the respiratory gas, however, is determined by the value of the predetermined parameter of oxygen concentration. Respiratory gas flow to the patient is triggered by fluctuations in the airway pressure, and can be controlled by operator-selected settings of positive end expiratory pressure (PEEP) and sensitivity. A continuous positive airway pressure (CPAP) is provided. "Sensitivity" is the pressure level below PEEP that a patient must produce in order to trigger a patient initiated mandatory breath, or a spontaneous breath from the ventilator. Mandatory breaths can be initiated by the ventilator according to the parameter settings, the operator, or the patient. Ventilator initiated breaths are delivered at a constant cycle interval determined by the operator selected value for respiratory rate. Operator initiated breaths are one time events whose occurrence the operator determines by manual controls. Patient initiated breaths are delivered whenever a patient effort reduces the airway pressure below PEEP by an amount equal to the operator selected value for sensitivity.

The invention also provides for precise control of oxygen concentration in the breathing gas. Gas measurement is based upon the ideal gas law, which states that the product of gas pressure and volume is proportional to the product of the number of moles and temperature of the gas, or

$$PV = NRT$$

where
P = absolute pressure of the gas;
V = gas container volume;
N = number of moles of gas;
R = the universal gas constant; and
T = absolute temperature of the gas.

Thus, the number of moles of gas in a fixed volume container can be determined indirectly by measuring the container volume, the gas pressure, and the gas temperature. A simple extension of this law implies that if gas is added or removed from the container, the pressure will change by a proportional amount;

$$\Delta P = \Delta NRT/V \qquad (1)$$

In addition, two ideal gases occupying the same space behave according to Dalton's law of partial pressures:

$$P = (N1 + N2)RT/V \qquad (2)$$

The mixing control algorithm is based upon equations 1 and 2. From (1), the number of gas molecules injected into the bottle is proportional to the measured pressure change:

$$\Delta N = \Delta PV/TR \qquad (3)$$

Thus, by proper calibration of the container, a pressure change measurement reads directly as moles of the fixed volume container, of gas injected, and can be used for gas mixed control. From (2) the number of moles of each gas in the respiratory gas containment vessel can be calculated from the mole ratios and pressure in the containment vessel:

$$NA = mix * PV/TR \qquad (4)$$

$$NO = (1 - mix) * PV/TR \qquad (5)$$

To fill the containment vessel to a desired mixture, the number of moles or molecules of each gas initially in the containment vessel is estimated by applying (4) and (5). The desired number of moles or molecules for each gas can be calculated by applying these equations for a desired mixture and pressure, and the pressure differential for each gas can be computed and converted to pressures using equation (3). Precise amounts of gas can be introduced into the containment vessel by sequentially injecting each gas to these pressure values. Similar calculations of the mixture and quantity of gas within the vessel may be performed for vessels with a volume which can change, either where the pressure is held fixed or where it is also subject to change.

This approach provides several significant advantages over other "batch type" mixing systems. Rapid mixing changes are possible since solenoids can be actuated independently, for example, to increase oxygen concentration, and an oxygen solenoid can be used exclusively for several cycles until the desired mixture is reached. In addition, high precision mixing is possible since a pressure transducer and a volume detector can provide extremely accurate, reliable gas pressure measurements, allowing for continuous mixture readjustment to correct for any variations that may result from mechanical valves. For example, if the fill solenoid valve happened to stick open for a brief time after it was signaled to close, the resulting pressure increase would be higher reflecting the additional amount of gas admitted. This variation is accomodated since the mix is determined from the measured pressure or volume change and so the amount of gas to admit in the next fill will be less.

Other aspects and advantages of the invention will become apparent from the following detailed description and the accompanying drawings, illustrating by way of example the features of the invention.

In the drawings:
FIGURE 1 is a simplified schematic diagram of the ventilator control system;
FIG. 2 is a detailed schematic diagram of the ventilator control system;
FIG. 3 is a graph of the air/oxygen mix control of example 1;
FIG. 4 is a graph of the receiver tank pressure for spontaneous ventilation (example 2);
FIG. 5 is a graph of receiver tank pressure for large mandatory breaths (example 3); and
FIG. 6 is a graph of receiver tank pressure for small mandatory breaths (example 4).

As is shown in the drawings for purposes of illustration, the invention is embodied in a ventilator control system having a separate inlet flow path for each pressurized gas, connected to an electrically operated solenoid valve for each gas. The outlet of each of the gas solenoid valves that make up the gas mixture is connected to a containment vessel, or tank, such that as each solenoid valve is energized, the gases are combined in the tank. The containment vessel is a gas receiver tank or bottle that is filled by the flow of gas from one or more inlet conduits. Pressure may be monitored by a precision pressure transducer connected to the tank, and a containment vessel outlet port is connected by a gas flow path to an electrically operated flow control valve. In the method of the invention, gas pressure or volume within the vessel is measured, and from this information and the Proportions of oxygen and other gases in the tank initially, the initial gas proportions are determined. The tank filling target pressures or volumes are determined, from a user defined parameter of oxygen concentration, and from the initial tank gas proportions, and each gas solenoid is actuated in turn to fill the tank from the initial level to the target levels. During the tank fill period the outlet flow control valve is closed, but once the gas proportioning fill cycle is complete, the outlet flow control is energized as required to provide the flow off precisely proportioned oxygen gas mixture to a patient. When the flow of gas out of the tank depletes the quantity of gas to a predetermined minimum, the cycle of proportional refilling is repeated.

In accordance with the present invention, there is provided a ventilator system for the delivery of respiratory gas to a patient comprising a containment vessel, inlet means connect the containment vessel in fluid communication with a source for respiratory gas; outlet means to connect the containment vessel in fluid communication with a patient; sensor means to sense a measure of the quantity respiratory gas in the containment vessel; inlet flow control means to control the flow of respiratory gas through the one inlet means in a predetermined manner responsive to the quantity of gas sensed in the containment vessel; and outlet flow control means to control the flow of respiratory gas through the outlet means in a predetermined manner responsive to changes in the quantity of gas sensed in the containment vessel over a period of time.

The present invention also provides for a method for delivering respiratory gas to a patient from a containment vessel; inlet means to connect the containment vessel in fluid communication with a source of respiratory gas; sensor means to sense a measure of the quantity of respiratory gas in the containment vessel; means to control the flow of respiratory gas through the inlet means; and outlet flow control means to control the flow of respiratory gas through the outlet means; comprising the steps of measuring the quantity of gas within the vessel; introducing the gas into the vessel through the inlet means until a predetermined quantity level in the vessel is reached; withdrawing the gas from the vessel through the outlet port; measuring the quantity of gas within the vessel periodically to determine a change in the quantity of gas over time; and controlling the withdrawing of gas from the vessel in a predetermined manner responsive to changes in the quantity of gas sensed in the containment vessel over a period of time.

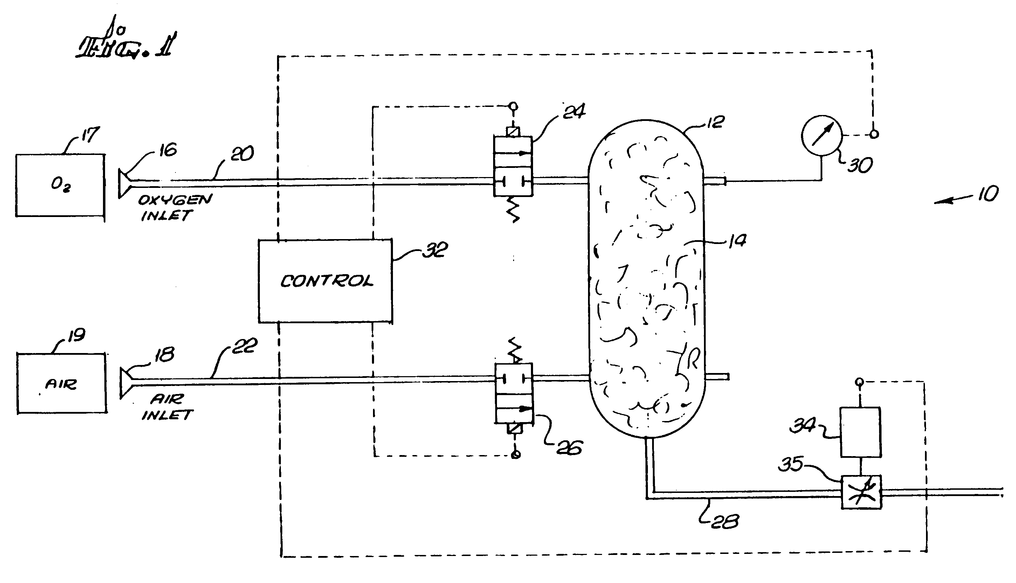

With reference to Fig. 1, the ventilator pneumatic control system 10 includes a receiver tank 12, which is in one currently preferred embodiment a rigid, fixed wall, pressure containment vessel, typically of approximately 2 liters in volume. Copper wool material 14 completely fills the interior volume of the tank, typically taking up to 2 percent of the tank volume. The copper wool, having a high specific heat value, ensures that the gas compression and decompression is relatively isothermal, during the filling and emptying cycles. Other similar material for reducing temperature fluctuations in the gas contained within the vessel may also be provided. The containment vessel may alternatively comprise a piston chamber, or a bellows-type of chamber, in which volume is adaptable to provide a variable capacity, with pressure being essentially constant, or in which volume and pressure are both variable, and both pressure and volume are monitored.

The receiver tank preferably has an oxygen inlet port 16 connected to an oxygen supply source 17. Similarly, the receiver tank also preferably includes an air inlet port 18 connected to an air supply source 19. The oxygen and air supply sources may simply be high pressure tanks of oxygen and air, and the air supply source may also be an air compressor. Other conventional sources of pressurized oxygen and air in hospital settings would also be appropriate. Alternatively, the air inlet line and oxygen inlet line may join to provide a premixing of the gases before they enter the containment vessel.

An oxygen supply conduit 29 preferably conveys oxygen from the oxygen inlet port to the tank, and an air supply conduit 22 conveys air to the receiver tank. The oxygen inlet line also has a solenoid valve 24 normally in a closed position, which can be energized by the electronic control system to supply pressurized oxygen to the receiver tank. Similarly, the air supply line includes a solenoid valve (26) which can be energized by the electronic control system to provide pressurized air to the receiver tank.

Also connected to the receiver tank are an outlet conduit 28 for supplying respiratory gas from the receiver tank to the patient, and a pressure sensing pressure transducer 30. The electronic control means 32, preferably including a microprocessor for controlling all of the functions of the ventilator control system, is connected to the oxygen solenoid valve, the air solenoid valve, the pressure sensor at the vessel, and an outlet solenoid valve control 34, which functions to control the adjustable inspiration servovalve 35 in the outlet conduit.

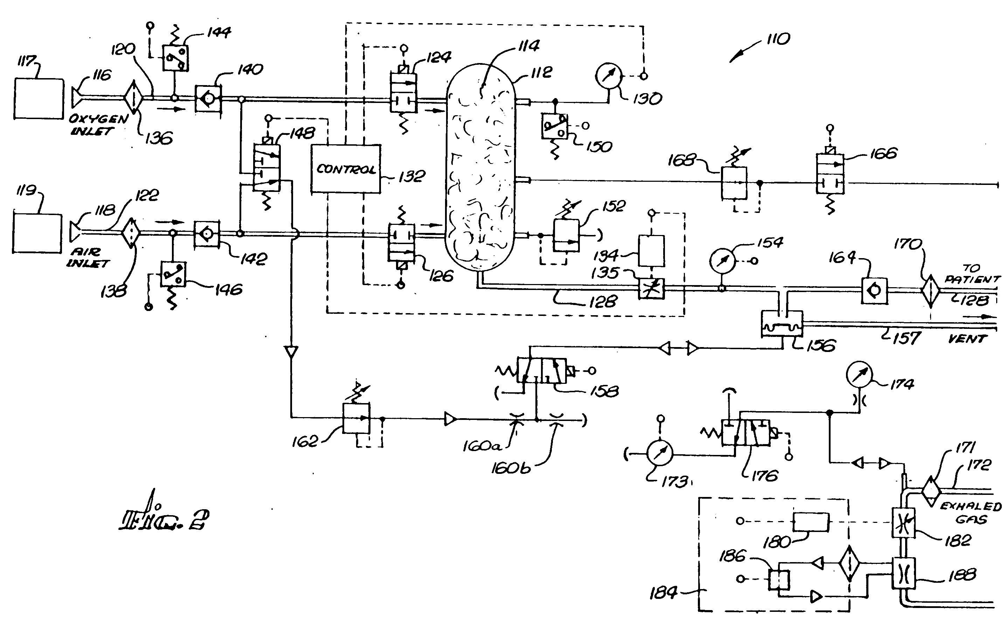

Referring to Fig. 2 those elements bearing reference numerals 110 through 134 correspond to elements bearing reference numerous 10 to 34, respectively. In the preferred mode of the invention, the oxygen inlet conduit also includes a filter 136 for the oxygen gas, and the air inlet conduit includes a filter 138 for the compressed air. Check valves 140 and 142 are also provided in the oxygen and air inlet conduits, and an oxygen pressure switch 144 and an air pressure switch 146 are connected near the gas inlet ports for low pressure gas supply monitoring. A crossover solenoid 148 is also preferably connected in line between the oxygen inlet conduit and the air inlet conduit to provide the ventilator with an alternate source of oxygen pressure should the normal respiratory gas system fail. The pressure switch 150 is connected to the receiver tank near the tank pressure sensor, and is used to electrically test the validity of the pressure transducer. A relief valve 152 is also connected to the receiver tank, and is used to relieve any over pressure conditions which may occur due to system or component failure.

Ventilator breathing gas flow is monitored by a second pressure sensor, the absolute pressure transducer 154 connected to the respiratory gas outlet conduit to the patient. Measurements made by the outflow absolute pressure transducer are used to determine patient system occlusion and atmosphere pressure and is also used to monitor validity of pressure readings in the patient breathing circuit. A safetey relief valve 156 is also provided on the respiratory gas flow line, which serves to vent excessive pressure in the respiratory gas outflow to the vent 157. A safety valve solenoid 158 and restrictors 160a and 160b are also connected in line with the safety regulator, connected in fluid communication with the crossover solenoid, to form the basics of the safety system to provide air to the patient should the normal ventilator air system fail. The safety relief valve thus serves the dual purpose of venting excess pressure, and admitting ambient air to the respiratory gas outflow line to the patient, if necessary. The safety system also includes the outlet line check valve 164.

A ventilator nebulizer pressure source is provided by the nebulizer solenoid 166, which receives its pressure supply from the nebulizer 168 connected directly to the receiver tank. Nebulizer operation will not vary the controlled volume delivery, or breathing gas, oxygen percentage.

The ventilator output gas flow is also filtered by the outlet line filter 170, and is transported to the patient by patient tubing (not shown). Exhaled gas is returned to the ventilator by tubes (not shown), connected to a water trap 171 at the exhalation valve inlet 172 in the patient breathing circuit. Additional ventilator components are connected by a patient pressure port at the exhalation valve inlet. These are a third pressure sensor 173, a manometer 174, used to monitor patient system pressure, and an autozero solenoid 176, used for electronically zeroing the patient breathing circuit pressure sensor 173.

An exhalation servovalve 180 is used to control the patient exhaled gas flow that flows to the room. The exhalation servovalve is a direct drive servovalve driven by an electric force motor and controlled by the ventilator electronic system. The force motor is connected mechanically to the exhalation valve 182 of the breathing circuit. The spirometer 184 is a two-part system that consists of the mass flow sensor 186 and the flow venturi 188. The spirometry monitored by the mass flow sensor and the flow venturi provides gas flow measurements to the ventilator electronic system for both the patient data display and ventilator status alarm elements. The electronic system controls all of the functioning and inputs of the ventilator pneumatic system, controlling oxygen inlet, air inlet and crossover solenoids, the inspiration servovalve and safety solenoid, as well as nebulizer solenoid and the autozero solenoid of the patient system pressure circuit. Each of the three pressure sensors and the flow sensor are also connected to the electronic system to provide data concerning the operation of the ventilator system.

Oxygen Air Proportioning.

In normal ventilator operation, bottle pressure is atmospheric when the power is first turned on. After completing power-up procedures, the ventilation algorithms begin execution.

First, bottle pressure is measured and prior oxygen concentration is retrieved from memory. Applying (4) and (5), the number of molecules of each gas type is estimated, (NA, NO). Then, mix and volume commands from the keyboard are used to compute "bottle-full" pressure and the desired number of molecules of each gas type (NAD, NOD). The estimated number is subtracted from the desired number to give the number of molecules of each type to inject. Finally, equation (2) is applied to convert these values into equivalent pressure change values ($\Delta$PA,$\Delta$PO).

After establishing a pressure charge value for each gas, target pressure levels are calculated. The first solenoid valve is opened to charge the bottle and then closed when the pressure reaches its first target pressure. After a short pause the bottle pressure is measured and recorded. Next, the second solenoid is opened and then closed when the second target pressure level is reached. After a short pause the bottle pressure is measured and recorded again.

With a mix established based on precise solenoid actuation, the true mix is now even more accurately estimated using equations (4) and (5) and the intermediate pressure measurements. This estimate replaces the former mix stored in memory.

Inspiration follows and gas from the bottle is delivered to the patient through the servovalve until the desired volume is delivered (mandatory breath) or the patient triggers end-of-inspiration (spontaneous). In either case, the mixing algorithm reengages.

At this point, the alogrithm follows two different procedures depending on the therapist-selected mode. In the spontaneous mode, the current bottle pressure is compared to a threshold. Only when it falls below this threshold will a new mixing cycle begin, and the bottle is filled completely. If it is above the threshold, mixing will not yet be performed. This threshold approach is used to reduce mixing inaccuracy due to pressure measurement errors.

In the mandatory mode, bottle pressure for the next cycle is estimated based on current pressure and the volume to be delivered to the patient on the next cycle. Specifically, the predicted pressure change due to a single volume delivery is subtracted from the current pressure. This estimate is then compared to a threshold and if it falls below, a new mixing cycle is performed as before. If not, mixing is skipped. This approach ensures that bottle pressure is always maintained above the threshold required for nebulizer operation.

EXAMPLE 1. AIR/OXYGEN MIX CONTROL

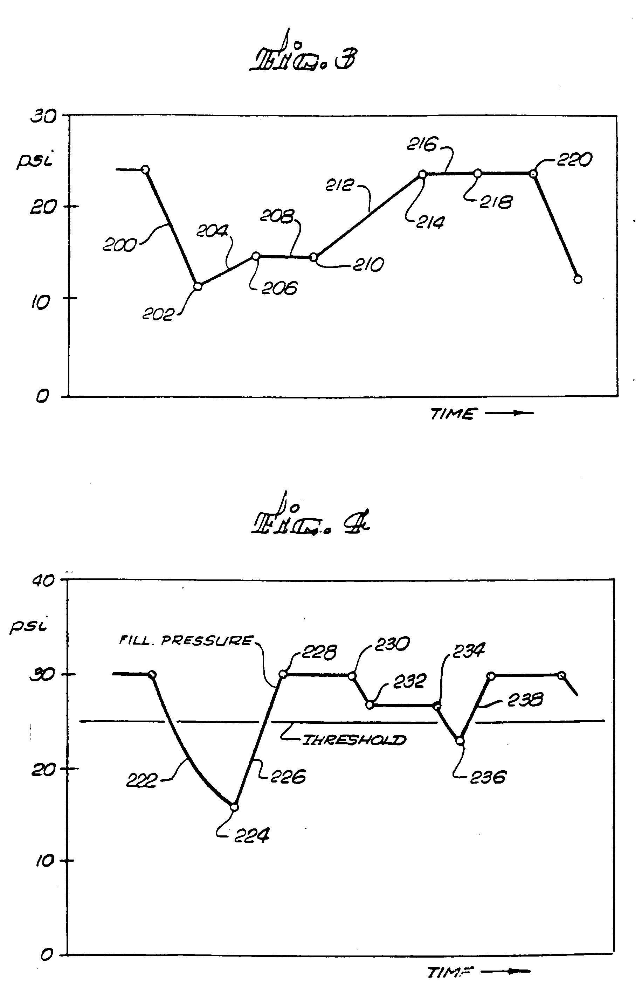

This example illustrates the events that compose a complete mix cycle. Figure 3 shows bottle pressure vs. time during the mix process. Marked events are:

1. Gas is delivered to patient through servovalve (200).
2. Gas delivery completed, servovalve is closed (202)
Bottle pressure is recorded.
Previous mix is retrieved from memory.
Current amount of each gas is estimated.
Keyboard entered mix command is retrieved from memory.
Desired bottle pressure is computed as follows:
    a. mandatory: add threshold, delivery pressure
    b. spontaneous: use maximum bottle pressure
Desired amount of each gas is calculated.
Amount of each gas to inject is obtained by subtraction.
3. Oxygen solenoid valve is opened (204). Pressure is monitored as oxygen is added.
4. Pressure reaches target for oxygen (206). Oxygen solenoid is closed.
5. A short pause allows pressure to settle (208).
6. Pressure is measured (210).
Pressure change due to oxygen is calculated and recorded.
7. Air solenoid valve is opened (212).
Pressure is monitored as air is added.
8. Pressure reaches target for air (214). Air solenoid is closed.
9. A short pause allows pressure to settle (216).

10. Pressure is measured (218).

Pressure change due to air is calculated and recorded. New mix is estimated from air and oxygen pressure changes measured.

11. Inspiration starts and gas begins to flow to the patient (220).

EXAMPLE 2. REFILL LOGIC FOR SPONTANEOUS VENTILATION.

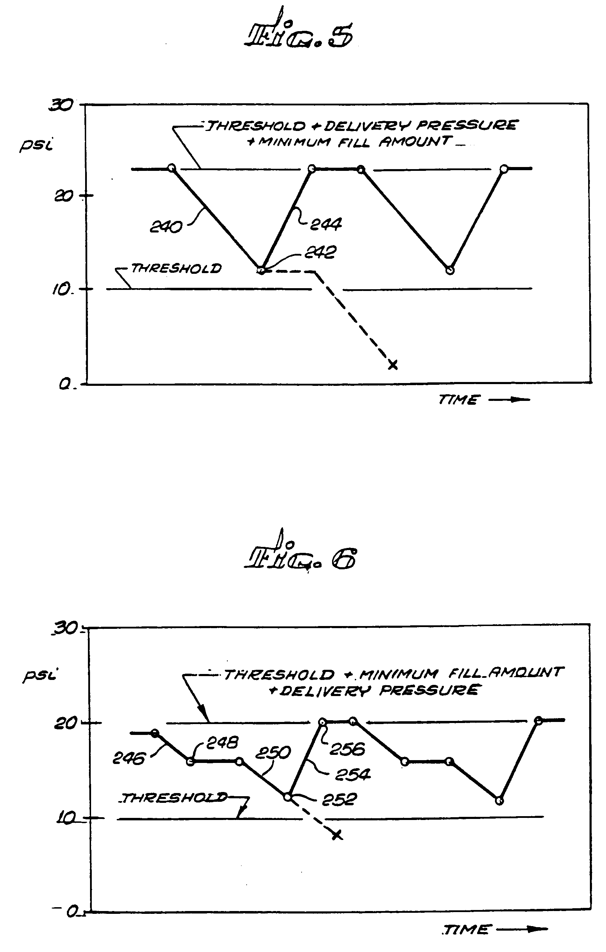

Spontaneous breaths are controlled by the patient and therefore are not predictable in size. The bottle must be fully recharged after every significant breath to ensure a large breath demand can be met in the next inspiration. In addition, mix accuracy depends largely on ensuring significant refill pressure change, therefore a threshold is used to enforce a minimum pressure charge. A typical pressure history is illustrated in Fig. 4:

1. Patient inspires (222).
2. Patient terminates inspiration (224).

Bottle pressure is recorded and compared to threshold.

Pressure is below threshold so proceed to refill.

Set target pressure to 30 psi.

3. Perform mix control as in Example 1 (226).
4. Pressure reaches 30 psi (228).

Mix control finished.

Wait for patient to inspire.

5. Patient inspires (230).
6. Patient terminates inspiration (232).

Bottle pressure is recorded and compared to threshold.

Pressure is above threshold so refill is not performed.

Wait for patient to inspire.

7. Patient inspires (234).
8. Patient terminates inspiration (236).

Bottle pressure is recorded and compared to threshold.

Pressure is below threshold so proceed to refill.

Set target pressure to 30 psi.

9. Perform mix control as in Example 1 (238).

EXAMPLE 3. REFILL LOGIC FOR LARGE MANDATORY BREATHS.

Mandatory ventilation provides predetermined breath size and so the bottle need not be completely refilled after each inspiration. This provides the opportunity to operate at lower pressures where the servovalve exhibits better linearity for improved flow control. However, a minimum pressure threshold is used to ensure sufficient nebulizer pressure. The bottle pressure is always maintained above the threshold. A typical bottle pressure history for this case is illustrated in Figure 5:

1. Gas is delivered to the patient (240).

2. Inspiration terminates as the desired volume is achieved (242).

Bottle pressure is recorded.

Forecast pressure after next breath.

Pressure forecast is below threshold, proceed to refill.

3. Perform mix control as in Example 1 (244).

Since the volumes in this example are large, the pressure will always fall below the threshold and so refill will occur after each breath.

EXAMPLE 4. REFILL LOGIC FOR SMALL MANDATORY BREATHS.

Small mandatory breaths produce small changes is bottle pressure and so refill should not occur after each inspiration. This prevents both mix inaccuracy and solenoid valve wear. A good example is illustrated in Figure 6:

1. Gas is delivered to the patient (246).
2. Inspiration terminates as the desired volume is achieved (248).

Bottle pressure is recorded.

Forecast pressure after next breath.

Forecast is above threshold so refill is not performed.

3. Gas is delivered to the patient (250).
4. Inspiration terminates as the desired volume is achieved (252).

Bottle pressure is recorded.

Forecast pressure after next breath.

Forecast is below threshold so proceed to refill.

5. Perform mix control as in Example 1 (254).
6. Mix control complete (256).

Wait for next breath.

Controlled volume Delivery.

For volume limited, mandatory ventilation, the operator determines parameters of tidal volume, and peak flow required, and enters these parameters into the electronic control system, to enable the ventilator system to deliver the desired breath. The drive gas pressure for the ventilator is stored in the receiver tank. During the inspiratory phase, the oxygen inlet solenoid valve and the air inlet solenoid valve remain closed, and the receiver tank pressure sensor continuously monitors pressure or volume levels within the receiver tank. At the start of inspiration, the initial pressure in the receiver tank is received from the pressure sensor, and entered into the microprocessor system memory, and the respiratory gas outlet solenoid servovalve is activated to regulate flow to the patient.

For peak flow control, an electronically differentiated pressure signal from the receiver tank pressure transducer is used to determine the gas flow rate out of the receiver tank. This dp/dt signal is used by the electronic control system to feed a control loop to the inspiration servovalve and maintain the desired set flow rate. The absolute pressure transducer in the outlet conduit, located downstream of the inspiration servovalve, provides measurements for atmospheric pressure, and for detection of breathing circuit occlusions.

A square wave form of ventilator flow is the standard method of controlled volume delivery. Volume control is accomplished by direct pressure measurement of the starting and end pressures in the receiver tank, during the inspiratory phase off ventilation. Volume is indirectly determined from the pressure change in the receiver tank, according to the ideal gas law equations.

The spontaneous breathing mode of the ventilator is an important function of the ventilator of the invention. The responsiveness of the ventilator to patient demand breathing determines the quality of breathing. The ventilator control system will implement a proportional servovalve controlled, spontaneous breathing mode with the servocontrol loop on the patient pressure sensor 173, located in the exhalation system flow path of the patient tubing for best response to patient demands.

In the foregoing description, it has been demonstrated that the system and method off the invention allow for control of the flow of respiratory gas into a pressure containment vessel in a predetermined manner responsive to pressure within the containment vessel and the mode of delivery of respiratory gas to a patient. It is also significant that the system and method of the invention allow for control of the outflow of respiratory gas to a patient in a predetermined manner responsive to changes in pressure sensed in the containment vessel over a period of time, and based upon the mode of delivery respiratory gas to a patient.

## Claims

1. A ventilator system (10, 110) for the delivery of respiratory gas to a patient comprising: a fixed wall containment vessel (12, 112); first and second inlet ports (16, 116; 18, 118) connected to said vessel (12, 112) with each said inlet port (16, 116; 18, 118) being in fluid communication with corresponding first and second separate sources (17, 117; 19, 119) of first and second respective component gases to be combined in a desired ratio to form said respiratory gas, with said first and second inlet ports (16, 116; 18, 118) having associated corresponding first and second inlet valves (24, 124; 26, 126) for individually controlling the flow of each said component gas through said inlet ports (16, 116; 18, 118) to said vessel (12, 112); outlet means (28, 128) to connect said vessel (12, 112) in fluid communication with the patient; sensor means (30, 130) to sense the pressure of said respiratory gas in the containment vessel (12, 112); inlet flow control means (32, 132) to sequentially control the flow of said first component gas into said containment vessel (12, 112) through said first inlet port (16, 116) while said second inlet port (18, 118) is closed, and to control the flow of said second component gas through said second inlet port (18, 118) while said first inlet port (16, 116) is closed, in said desired ratio responsive to the pressure of gas sensed in the containment vessel, said control means including means for determining a desired pressure change value for each said component gas in said containment vessel (12, 112) corresponding to the number of moles of each said component gas required to fill said vessel (12, 112) with said desired ratio of said component gases, by determining an estimated number of molecules in said vessel (12, 112) from sensed pressure in the vessel (12, 112) and a prior first component gas concentration, and subtracting the estimated number of molecules from a desired number of molecules in said vessel (12, 112), and means for determining an actual ratio of said component gases in said respiratory gas in said vessel (12, 112) by determining the number of moles of each of said component gases in said vessel (12, 112) based upon sensed changes in pressure of each of said component gases introduced into said containment vessel (12, 112); and outlet flow control means (32, 132) including microprocessor or electronic means electrically connected to said sensor means (30, 130) for controlling the outflow of said respiratory gas through the outlet means (28, 128) characterized by:

   said sensor means (30, 130) including means for electronically differentiating pressure sensed in said containment vessel over a period of time to generate a differentiated pressure signal representing an outflow rate of change of pressure in said containment vessel over a period of time, and said outlet flow control means (32, 132) controlling said outflow of respiratory gas responsive to such differentiated pressure signal based upon the rate of change in the pressure of gas sensed in the containment vessel (12, 112) over a period of time.

2. A system as claimed in claim 1, wherein said outlet flow control means (32, 132) comprises means for determining the rate of outflow of said respiratory gas from said vessel (12, 112) responsive to said differentiated pressure signal from said sensor means (30, 130), output comparison means (32, 132) for comparing said rate of outflow with a predetermined reference outflow value adapted to generate an error signal, and valve means (34, 35; 134, 135) in fluid communication with said outlet means (28, 128) operatively connected to said output comparison means (32, 132) to receive said error signal, and adapted to adjust said outflow to said predetermined outflow value in response to said signal.

3. A system as claimed in claim 1, wherein said inlet flow control means (32, 132) includes fill control means (32, 132) for comparing the pressure of gas in said vessel with a predetermined threshold pressure, responsive to said pressure of gas sensed by said sensor means (30, 130), and adapted to generate a fill signal when the pressure of gas in said vessel (12, 112) falls below said threshold pressure value, said first and second inlet valves (16, 116; 18, 118) being operatively connected to said fill control means (32, 132) for receiving said fill signal.

4. A system as claimed in claim 1, wherein said inlet flow control means (32, 132) includes means (32, 132) for determining a predicted pressure of gas in said vessel after a predicted next inhalation by the patient, based upon sensed rate of change of pressure in said vessel (12, 112) and fill control means (32, 132) for comparing said predicted pressure of gas with a predetermined threshold value.

5. A system as claimed in any one of the preceding claims, wherein said vessel contains thermally conductive means (14, 114) for reducing temperature fluctuation of gas contained within said vessel (12, 112), said means for reducing temperature fluctuation (14, 114) being dispersed throughout the interior volume of said vessel (12, 112).

6. A system as claimed in claim 5, wherein said means for reducing temperature fluctuation (14, 114) comprises copper wool.

7. A method for controlling the delivering of respiratory gas to a patient from a ventilator system comprising a fixed wall containment vessel (12, 112) having first and second inlet ports (16, 116; 18, 118), each of said inlet ports (16, 116; 18, 118) connecting the vessel (12, 112) in fluid communication with first and second separate sources (17, 117; 19, 119) of respective first and second component gases to be combined in said vessel (12, 112) in a desired ratio to form said respiratory gas, said respiratory gas in said vessel (12, 112) having an initial known ratio of said component gases; outlet means (28, 128) to connect the vessel (12, 112) in fluid communication with the patient; sensor means (30, 130) to sense the pressure of said respiratory gas in the containment vessel (12, 112); means (32, 132) to sequentially control the flow of said component gases through said inlet ports (16, 116; 18, 118); and output flow control means (34, 35; 134, 135) to control the flow of said respiratory gas through said outlet means (28, 128), said method comprising the steps of: measuring the pressure of gas within said vessel (12, 112) following inhalation by the patient; determining pressure increase values for said first and second component gases in said vessel (12, 112) corresponding to the number of molecules of each said component gas required to fill said vessel (12, 112) with said desired ratio of said component gases to a desired fill pressure by determining an estimated number of molecules in said vessel (12, 112) from sensed pressure in the vessel (12, 112) and a prior first component gas concentration, and subtracting the estimated number of molecules from a desired number of molecules in said vessel (12, 112); sequentially filling said vessel (12, 112) with said component gases to said corresponding pressure increase values while measuring pressure in said containment vessel (12, 112), by introducing said first component gas into said vessel (12, 112) through said first inlet port (16, 116) while said second inlet port (18, 118) is closed, and said second component gas through said second inlet port (18, 118) while said first inlet (16, 116) is closed according to said pressure increase values until said fill pressure of gas in said vessel (12, 112) is reached; measuring an actual pressure increase in said containment vessel (12, 112) for each said component gas introduced into said vessel (12, 112); determining the ratio of said component gases in said containment vessel (12, 112) by determining the number of moles of each of said component gases in said vessel (12, 112) based upon said initial known ratio of component gases in said vessel (12, 112), said total pressure and said measured actual pressure changes for each said component gas introduced into said vessel (12, 112); withdrawing said gas from said vessel (12, 112) through said outlet means (28, 128); measuring an outflow rate of change in the pressure of gas in the vessel (12, 112) over time;

and controlling (32, 132) said withdrawing of gas from said vessel (12, 112), characterized by:

generating a differentiated pressure signal representing the outflow rate of change of pressure in said containment vessel (12, 112) over a period of time, and said controlling of withdrawing of gas being responsive to said differentiated pressure signal based upon the rate of change in the pressure of gas sensed in said containment vessel (12, 112) over a period of time.

8. A method as claimed in claim 7 wherein said step of controlling (32, 132) said withdrawing of gas comprises comparing (32, 132) said change in the pressure of gas with a predetermined reference outflow value and generating (32, 132) an error signal representing the difference between said change in the pressure of gas and said reference outflow value; and

adjusting (32, 132) said outflow in said outlet in response to said error signal to reduce the difference between said rate of change in the pressure of gas and said reference outflow value.

9. A method as claimed in claim 7, further including the steps of:

comparing (32, 132) said measured pressure of gas in said vessel with a predetermined threshold value; and

introducing said first and second component gases into said vessel (12, 112) in said desired ratio when the pressure in said vessel (12, 112) falls below said threshold value and until the total pressure of said gas in said vessel (12, 112) reaches a predetermined upper limit.

10. A method as claimed in claim 7, further including the steps of:

determining (32, 132) a predicted pressure in said vessel (12, 112) after a predicted next inhalation by the patient, based upon said sensed rate of change in pressure in said vessel (12, 112);

comparing (32, 132) said predicted pressure with a predetermined threshold pressure value; and

filling said vessel (12, 112) with said gas when said predicted pressure falls below said predetermined threshold pressure value.

11. A method as claimed in claim 7, wherein said respiratory gas is withdrawn from said vessel (12, 112) for a predetermined period of time according to a withdrawal flow command, and further including the steps of:

a) subtracting (32, 132) said measured outflow change in pressure from a desired pressure change to obtain a gas pressure change error in the amount of gas delivered in a gas delivery;

b) integrating (32, 132) said gas pressure change error for each said delivery to determine an integral error in the amount of gas delivered;

c) setting (32, 132) said withdrawal flow command for the next withdrawal to said desired pressure change plus an amount proportional to the above integral error; and

d) repeating steps a) - c) for a subsequent delivery of a volume of gas.

12. A method as claimed in claim 7, further comprising the steps of:

measuring (32, 132) the volume of the flow in said outlet means based upon changes in the pressure (30, 130) of said gas in said containment vessel (12, 112) following inhalation by the patient;

comparing (32, 132) said volume of flow in said outlet means (28, 128) with a predetermined outlet lower threshold level and with a predetermined outlet upper threshold level;

initiating outflow (34, 35; 134, 135) of said gas through said outlet means (28, 128) when said outlet flow volume falls below said lower threshold level; and stopping outflow (34, 35; 134, 135) of said gas through said outlet means (28, 128) when said volume of outlet flow rises above said predetermined upper threshold level.

**Patentansprüche**

1. Beatmungssystem (10, 110) zur Zufuhr von Atemgas zu einem Patienten, umfassend: ein Druckgefäß (12, 112) mit fester Wandung; erste und zweite Einlaßöffnungen (16, 116; 18, 118), welche mit dem Gefäß (12 112) verbunden sind, wobei jede Einlaßöffnung (16, 116; 18, 118) in Fluidverbindung mit entsprechenden ersten und zweiten gesonderten Quellen (17, 117; 19, 119) jeweiliger erster und zweiter, zur Bildung des Atemgases in einem gewünschten Verhältnis zu vereinender Komponentengase steht, wobei den ersten und zweiten Einlaßöffnungen (16, 116; 18, 118) entsprechende erste und zweite Einlaßventile (24, 124; 26, 126) zugeordnet sind zum individuellen Steuern des Flusses jedes Komponentengases durch die Einlaßöffnungen (16, 116; 18, 118) zu dem Gefäß (12, 112); Auslaßmittel (28, 128), um das Gefäß (12, 112) mit dem Patienten in Fluidverbindung zu setzen; Sensormittel (30, 130), um den Druck des Atemgases in dem Druckgefäß (12, 112) zu erfassen; Einlaßfluß-Steuermittel (32, 132), um in Antwort auf den in dem Druckgefäß erfaßten Gasdruck in dem gewünschten Verhältnis den Fluß des ersten Komponentengases durch die erste Einlaßöffnung (16, 116) in das Druckgefäß (12, 112) zu steuern, während die zweite Einlaßöffnung (18, 118) geschlossen ist, und nachfolgend den Fluß des zweiten Komponentengases durch die zweite Einlaßöffnung (18, 118) zu steuern, während die erste Einlaßöffnung (16, 116) geschlossen ist, wobei das Steuermittel umfaßt: Mittel, um gemäß der Molanzahl jedes Komponentengases für jedes Komponentengas in dem Druckgefäß (12, 112) einen zum Befüllen des Gefäßes (12, 112) mit dem gewünschten Verhältnis der Komponentengase erforderlichen, gewünschten Druckänderungswert zu bestimmen, indem aus dem erfaßten Druck in dem Gefäß (12, 112) und einer früheren Konzentration des ersten Komponentengases eine geschätzte Anzahl von Molekülen in dem Gefäß (12, 112) bestimmt wird, und die geschätzte Anzahl von Molekülen von einer gewünschten Anzahl von Molekülen in dem Gefäß (12, 112) abgezogen wird, sowie Mittel, um ein tatsächliches Verhältnis der Komponentengase in dem Atemgas in dem Gefäß (12, 112) zu bestimmen, indem die Molanzahl jedes der Komponentengase in dem Gefäß (12, 112) auf Grundlage erfaßter Druckänderungen jedes der in das Druckgefäß (12, 112) eingeleiteten Komponentengase bestimmt wird; und einen Mikroprozessor oder elektronische Mittel umfassende Auslaßfluß-Steuermittel (32, 132), die elektrisch mit den Sensormitteln (30, 130) verbunden sind, um den Ausfluß des Atemgases durch die Auslaßmittel (28, 128) zu steuern, dadurch gekennzeichnet,

daß die Sensormittel (30, 130) Mittel umfassen, um den in dem Druckgefäß über eine Zeitdauer erfaßten Druck elektronisch zu differenzieren, um ein differenziertes Drucksignal zu erzeugen, welches eine Ausfluß-Änderungsrate des Drucks in dem Druckgefäß über eine Zeitdauer darstellt, und daß die Auslaßfluß-Steuermittel (32, 132) den Ausfluß von Atemgas in Antwort auf dieses differenzierte Drucksignal auf Grundlage der Änderungsrate des in dem Druckgefäß (12, 112) über eine Zeitdauer erfaßten Gasdrucks steuern.

2. System nach Anspruch 1, bei welchem die Auslaßfluß-Steuermittel (32, 132) umfassen: Mittel, um die Ausflußrate des Atemgases aus dem Gefäß (12, 112) in Antwort auf das differenzierte Drucksignal von den Sensormitteln (30, 130) zu bestimmen, Ausgabevergleichsmittel (32, 132), um die Ausflußrate mit einem vorbestimmten Referenzausflußwert zu vergleichen, welche zur Erzeugung eines Fehlersignals ausgebildet sind, sowie Ventilmittel (34, 35; 134, 135), die mit den Auslaßmitteln (28, 128) in Fluidverbindung stehen und mit den Ausgabevergleichsmitteln (32, 132) betriebsmäßig verbunden sind, um das Fehlersignal zu empfangen, und dazu ausgelegt sind, den Ausfluß in Antwort auf dieses Signal auf den vorbestimmten Ausflußwert einzustellen.

3. System nach Anspruch 1, bei welchem die Einlaßfluß-Steuermittel (32, 132) Befüllungs-Steuermittel (32, 132) umfassen, um in Antwort auf den durch die Sensormittel (30, 130) erfaßten Gasdruck den Gasdruck in dem Gefäß mit einem vorbestimmten Schwellendruck zu vergleichen, und ausgelegt sind, um ein Befüllungssignal zu erzeugen, wenn der Gasdruck in dem Gefäß (12, 112) unter den Druckschwellenwert abfällt, wobei die ersten und zweiten Einlaßventile (16, 116; 18, 118) mit den Befüllungs-Steuermitteln (32, 132) betriebsmäßig verbunden sind, um das Befüllungssignal zu empfangen.

4. System nach Anspruch 1, bei welchem die Einlaßfluß-Steuermittel (32, 132) Mittel (32, 132) umfassen, um einen vorhergesagten Gasdruck in dem Gefäß nach einer vorhergesagten nächsten Inhalation durch den Patienten auf Grundlage der erfaßten Druckänderungsrate in dem Gefäß (12, 112) zu bestimmen, sowie Befüllungs-Steuermittel (32, 132), um den vorhergesagten Gasdruck mit einem vorbestimmten Schwellenwert zu vergleichen.

5. System nach einem der vorhergehenden Ansprüche, bei welchem das Gefäß thermisch leitfähige Mittel (14, 114) enthält, um Temperaturschwankungen des in dem Gefäß (12, 112) enthaltenen Gases zu mindern, wobei die Mittel (14, 114) zum Mindern von Temperaturschwankungen über das gesamte Innenvolumen des Gefäßes (12, 112) verteilt sind.

6. System nach Anspruch 5, bei welchem die Mittel (14, 114) zum Mindern von Temperaturschwankungen Kupferwolle umfassen.

7. Verfahren zum Steuern der Zufuhr von Atemgas zu einem Patienten aus einem Beatmungssystem, umfassend ein Druckgefäß (12, 112) mit fester Wandung mit ersten und zweiten Einlaßöffnungen (16, 116; 18, 118), wobei jede Einlaßöffnung (16, 116; 18, 118) das Gefäß (12, 112) mit ersten und zweiten gesonderten Quellen (17, 117, 19, 119) jeweiliger erster und zweiter, in dem Gefäß (12, 112) in einem gewünschten Verhältnis zur Bildung des Atemgases zu vereinender Komponentengase in Fluidverbindung setzt, wobei das Atemgas in dem Gefäß (12, 112) ein bekanntes Anfangsverhältnis der Komponentengase aufweist; Auslaßmittel (28, 128), um das Gefäß (12, 112) mit dem Patienten in Fluidverbindung zu setzen; Sensormittel (30, 130), um den Druck des Atemgases in dem Druckgefäß (12, 112) zu erfassen; Mittel (32, 132), um den Fluß der Komponentengase durch die Einlaßöffnungen (16, 116; 18, 118) aufeinanderfolgend zu steuern; und Ausgangsfluß-Steuermittel (34, 35; 134, 135), um den Fluß des Atemgases durch die Auslaßmittel (28, 128) zu steuern, das Verfahren umfassend die Schritte: Messen des Gasdrucks in dem Gefäß (12, 112) nach Inhalation durch den Patienten; Bestimmen der Druckanstiegswerte für die ersten und zweiten Komponentengase in dem Gefäß (12, 112) entsprechend der Anzahl von Molekülen jedes Komponentengases, die erforderlich ist, um das Gefäß (12, 112) mit dem gewünschten Verhältnis der Komponentengase auf einen gewünschten Befüllungsdruck zu füllen, indem eine geschätze Anzahl von Molekülen in dem Gefäß (12, 112) aus dem erfaßten Druck in dem Gefäß (12, 112) und einer früheren Konzentration des ersten Komponentengases bestimmt wird, und die geschätzte Anzahl von Molekülen von einer gewünschten Anzahl von Molekülen in dem Gefäß (12, 112) subtrahiert wird; aufeinanderfolgendes Befüllen des Gefäßes (12, 112) mit den Komponentengasen auf die entsprechenden Druckanstiegswerte, während Druckmessung in dem Druckgefäß (12, 112), indem gemäß den Druckanstiegswerten das erste Komponentengas in das Gefäß (12, 112) durch die erste Einlaßöffnung (16, 116) eingeleitet wird, während die zweite Einlaßöffnung (18, 118) geschlossen ist, und das zweite Komponentengas durch die zweite Einlaßöffnung (18, 118) eingeleitet wird, während der erste Einlaß (16, 116) geschlossen ist, bis der Gasbefüllungsdruck in dem Gefäß (12, 112) erreicht ist; Messen eines tatsächlichen Druckanstiegs in dem Druckgefäß (12, 112) für jedes in das Gefäß (12, 112) eingeleitete Komponentengas; Bestimmen des Verhältnisses der Komponentengase in dem Druckgefäß (12, 112) durch Bestimmen der Molanzahl jedes der Komponentegase in dem Gefäß (12, 112) auf Grundlage des bekannten Anfangsverhältnisses der Komponentengase in dem Gefäß (12, 112), des Gesamtdrucks und der gemessenen tatsächlichen Druckänderungen für jedes in das Gefäß (12, 112) eingeleitete Komponentengas; Entnehmen des Gases aus dem Gefäß (12, 112) durch die Auslaßmittel (28, 128); Messen einer Ausflußänderungsrate des Gasdrucks in dem Gefäß (12, 112) über die Zeit; und Steuern (32, 132) der Gasentnahme aus dem Gefäß (12, 112), gekennzeichnet durch:

Erzeugen eines differenzierten Drucksignals, welches die Ausflußänderungsrate des Drucks in dem Druckgefäß (12, 112) über eine Zeitdauer darstellt, und wobei das Steuern der Gasentnahme in Antwort auf das differenzierte Drucksignal auf Grundlage der Änderungsrate des in dem Druckgefäß (12, 112) erfaßten Gasdrucks über eine Zeitdauer erfolgt.

8. Verfahren nach Anspruch 7, bei welchem der Schritt des Steuerns (32, 132) der Gasentnahme umfaßt Vergleichen (32, 132) der Gasdruckänderung mit einem vorbestimmten Referenzausflußwert und Erzeugen (32, 132) eines Fehlersignals, das die Differenz zwischen der Gasdruckänderung und dem Referenzausflußwert darstellt; und Einstellen (32, 132) des Ausflusses in dem Auslaß in Antwort auf das Fehlersignal, um die Differenz zwischen der Gasdruckänderungsrate und dem Referenzausflußwert zu vermindern.

9. Verfahren nach Anspruch 7, ferner umfassend die Schritte:

Vergleichen (32, 132) des gemessenen Gasdrucks in dem Gefäß mit einem vorbestimmten Schwellenwert; und

Einleiten der ersten und zweiten Komponentengase in das Gefäß (12, 112) in dem gewünschten Verhältnis, wenn der Druck in dem Gefäß (12, 112) unter den Schwellenwert abfällt, und bis der Gesamtdruck des Gases in dem Gefäß (12, 112) einen vorbestimmten oberen Grenzwert erreicht.

10. Verfahren nach Anspruch 7, ferner umfassend die Schritte:

Bestimmen (32, 132) eines vorhergesagten Drucks in dem Gefäß (12, 112) nach einer vorhergesagten nächsten Inhalation durch den Patienten auf Grundlage der erfaßten Druckänderungsrate in dem Gefäß (12, 112);

Vergleichen (32, 132) des vorhergesagten Drucks mit einem vorbestimmten Druckschwellenwert; und

Befüllen des Gefäßes (12, 112) mit dem Gas, wenn der vorhergesagte Druck unter den vorbestimmten Druckschwellenwert abfällt.

11. Verfahren nach Anspruch 7, bei welchem das Atemgas aus dem Gefäß (12, 112) gemäß einem Entnahmeflußbefehl für eine vorbestimmte Zeitdauer entnommen wird, und ferner umfassend die Schritte:

   a) Subtrahieren (32, 132) der gemessenen Ausflußänderung des Drucks von einer erwünschten Druckänderung, um einen Gasdruck-Änderungsfehler in der in einer Gaszufuhr zugeführten Gasmenge zu erhalten;

   b) Integrieren (32, 132) des Gasdruck-Änderungsfehlers für jede Zufuhr, um einen integralen Fehler in der zugeführten Gasmenge zu bestimmen;

   c) Festsetzen (32, 132) des Entnahmeflußbefehls für die nächste Entnahme auf die gewünschte Druckänderung zuzüglich eines Betrags, der dem vorstehenden integralen Fehler proportional ist; und

   d) Wiederholen der Schritte a) - c) für eine nachfolgende Zufuhr eines Gasvolumens.

12. Verfahren nach Anspruch 7, ferner umfassend die Schritte:

   Messen (32, 132) des Flußvolumens in den Auslaßmitteln auf Grundlage der Druckänderungen (30, 130) des Gases in dem Druckgefäß (12, 112) nach Inhalation durch den Patienten;

   Vergleichen (32, 132) des Flußvolumens in den Auslaßmitteln (28, 128) mit einem vorbestimmten unteren Auslaßschwellenwert und mit einem vorbestimmen oberen Auslaßschwellenwert;

   Einleiten des Ausflusses (34, 35; 134, 135) des Gases durch die Auslaßmittel (28, 128), wenn das Auslaßflußvolumen unter den unteren Schwellenwert abfällt; und

   Anhalten des Ausflusses (34, 35; 134, 135) des Gases durch die Auslaßmittel (28, 128), wenn das Volumen des Auslaßflusses über den vorbestimmten oberen Schwellenwert ansteigt.

**Revendications**

1. Système de respirateur (10, 110) pour délivrer un gaz respiratoire à un patient comprenant :
une cuve réservoir à parois fixes (12, 112) ;
une première et une seconde ouvertures d'entrée (16, 116 ; 18, 118) reliées à ladite cuve (12, 112), chacune desdites ouvertures d'entrée (16, 116 ; 18, 118) étant en communication pour les fluides avec une première et une seconde sources distinctes correspondantes (17, 117 ; 19, 119) des premier et second gaz composants respectifs qui doivent être combinés dans un rapport souhaité pour former ledit gaz respiratoire, lesdites première et seconde ouvertures d'entrée (16, 116 ; 18, 118) étant associées à une première et une seconde vannes d'entrée correspondantes (24, 124 ; 26, 126) pour commander individuellement le flux de chacun desdits gaz composants traversant lesdites ouvertures d'entrée (16, 116 ; 18, 118) vers ladite cuve (12, 112) ; des moyens de sortie (28, 128) pour connecter ladite cuve (12, 112) avec le patient en communication pour les fluides ; des moyens détecteurs (30, 130) pour détecter la pression dudit gaz respiratoire dans ladite cuve réservoir (12, 112) ; des moyens de commande du flux d'entrée (32, 132) pour commander séquentiellement le flux dudit premier gaz composant vers l'intérieur de ladite cuve réservoir (12, 112) à travers ladite première ouverture d'entrée (16, 116), tandis que ladite seconde ouverture d'entrée (18, 118) est fermée, et pour commander le flux dudit second gaz composant à travers ladite seconde ouverture d'entrée (18, 118), tandis que ladite première ouverture d'entrée (16, 116) est fermée, dans ledit rapport souhaité en réponse à la pression du gaz détectée dans la cuve réservoir, lesdits moyens de commande comprenant des moyens pour déterminer une valeur souhaitée de changement de pression pour chacun desdits gaz composants dans ladite cuve réservoir (12, 112) correspondant au nombre de moles de chacun desdits gaz composants nécessaire pour remplir ladite cuve (12, 112) avec ledit rapport souhaité desdits gaz composants en déterminant un nombre estimatif de molécules dans ladite cuve (12, 112) d'après la pression détectée dans la cuve (12, 112) d'après une concentration antérieure du premier gaz composant, et en retranchant le nombre estimatif de molécules d'un nombre désiré de molécules dans ladite cuve (12, 112), et des moyens afin de déterminer un rapport réel desdits gaz composants dans ledit gaz respiratoire contenu dans ladite cuve (12, 112) en déterminant le nombre de moles de chacun desdits gaz composants dans ladite cuve (12, 112) d'après les variations détectées de pression de chacun desdits gaz composants introduits dans ladite cuve réservoir (12, 112) ; et des moyens de commande du flux de sortie (32, 132) comportant un microprocesseur ou des moyens électroniques, électriquement reliés auxdits moyens détecteurs (30, 130), pour commander le flux de sortie dudit gaz respiratoire à travers les moyens de sortie (28, 128), caractérisé par :

   le fait que lesdits moyens détecteurs (30, 130) comprennent des moyens pour différencier par un procédé électronique la pression détectée dans ladite cuve réservoir pendant une certaine période de temps pour former un signal de pression différencié représentant une vitesse de variation de la pression due au flux sortant dans ladite cuve réservoir pendant une certaine période de temps, et lesdits moyens de commande du flux de sortie (32, 132) commandant ledit flux de sortie du gaz respiratoire en réponse à ce signal différencié de pression en fonction de la vitesse de variation de la pression du gaz détectée dans la cuve réservoir (12, 112) pendant une certaine période de temps.

2. Système selon la revendication 1, dans lequel lesdits moyens de commande du flux de sortie (32, 132) comprennent des moyens pour déterminer la vitesse du flux dudit gaz respiratoire sortant de la cuve (12, 112), qui répondent audit signal de pression différencié délivré par lesdits moyens détecteurs (30, 130), des moyens de comparaison de sortie (32, 132) pour comparer ladite vitesse du flux de sortie à une valeur de référence déterminée d'avance du flux de sortie, adaptés à former un signal d'erreur, et des moyens de vannes (34, 35 ; 134, 135) en communication pour les fluides avec lesdits moyens de sortie (28, 128), fonctionnellement connectés auxdits moyens de comparaison de sortie (32, 132) pour recevoir ledit signal d'erreur et adaptés pour ajuster ledit flux de sortie à ladite valeur déterminée d'avance du flux de sortie en réponse audit signal.

3. Système selon la revendication 1, dans lequel lesdits moyens de commande du flux d'entrée (32, 132) comprennent des moyens de commande de remplissage (32, 132) pour comparer la pression du gaz à l'intérieur de ladite cuve a un seuil de pression déterminé d'avance, répondant à ladite pression du gaz détectée par lesdits moyens détecteurs (30, 130) et adaptés pour former un signal de remplissage quand la pression du gaz à l'intérieur de ladite cuve (12, 112) descend au-dessous de ladite valeur du seuil de pression, lesdites première et seconde vannes d'entrée (16, 116 ; 18, 118) étant fonctionnellement reliées auxdits moyens de commande de remplissage (32, 132) pour recevoir ledit signal de remplissage.

4. Système selon la revendication 1, dans lequel lesdits moyens de commande du flux d'entrée (32, 132) comprennent des moyens (32, 132) pour déterminer une pression du gaz dans ladite cuve déterminée d'avance après une prochaine inhalation prévue par le patient, d'après la vitesse détectée de variation de la pression dans ladite cuve (12, 112), et des moyens de commande de remplissage (32, 132) pour comparer ladite pression de gaz prévue à une valeur de seuil déterminée d'avance.

5. Système selon l'une quelconque des revendications précédentes, dans lequel ladite cuve contient des moyens conducteurs thermiques (14, 114) pour réduire les fluctuations de température du gaz contenu dans ladite cuve (12, 112) , lesdits moyens pour réduire les fluctuations de température (14, 114) étant dispersés dans tout le volume intérieur de ladite cuve (12, 112).

6. Système selon la revendication 5, dans lequel lesdits moyens pour réduire les fluctuations de température (14, 114) sont constitués par de la laine de cuivre.

7. Procédé pour commander la délivrance d'un gaz respiratoire à un patient à partir d'un système de respirateur comprenant : une cuve réservoir à parois fixes (12, 112) ayant une première et une seconde ouvertures d'entrée (16, 116 ; 18, 118), chacune desdites ouvertures d'entrée (16, 116 ; 18, 118) reliant la cuve (12, 112) en communication pour les fluides avec une première et une seconde sources distinctes (17, 117 ; 19, 119) des premier et second gaz composants respectifs devant être combinés dans ladite cuve (12, 112) avec un rapport souhaité pour constituer ledit gaz respiratoire, ledit gaz respiratoire contenu dans ladite cuve (12, 112) ayant un rapport initial connu desdits gaz composants ; des moyens de sortie (28, 128) pour connecter la cuve (12, 112) en communication pour les fluides avec le patient ; des moyens détecteurs (30, 130) pour détecter la pression dudit gaz respiratoire à l'intérieur de la cuve réservoir (12, 112) ; des moyens (32, 132) pour commander séquentiellement le flux desdits gaz composants à travers lesdites ouvertures d'entrée (16, 116 ; 18, 118) , et des moyens de commande du flux de sortie (34, 35 ; 134, 135) pour commander le flux dudit gaz respiratoire à travers lesdits moyens de sortie (28, 128), ledit procédé comprenant les étapes suivantes : mesurer la pression du gaz à l'intérieur de ladite cuve (12, 112) après inhalation par le patient ; déterminer les valeurs de l'augmentation de pression dudit premier et dudit second gaz composants dans ladite cuve (12, 112), correspondant au nombre de molécules de chacun desdits gaz composants nécessaire pour remplir ladite cuve (12, 112) avec ledit rapport souhaité desdits gaz composants jusqu'à une pression de remplissage souhaitée en déterminant un nombre estimatif de molécules dans ladite cuve (12, 112) d'après la pression détectée dans la cuve (12, 112) et d'après une première concentration antérieure des gaz composants, et en retranchant le nombre estimatif de molécules d'un nombre souhaité de molécules dans ladite cuve (12, 112) ; remplir séquentiellement ladite cuve (12, 112) avec lesdits gaz composants jusqu'auxdites valeurs correspondantes d'augmentation de la pression tout en mesurant la pression dans ladite cuve réservoir (12, 112), en introduisant ledit premier gaz composant dans ladite cuve (12, 112) à travers ladite première ouverture d'entrée (16, 116), tandis que ladite seconde ouverture d'entrée (18, 118) est fermée, et ledit second gaz composant à travers ladite seconde ouverture d'entrée (18, 118), tandis que ladite première entrée (16, 116) est fermée selon lesdites valeurs d'augmentation de pression jusqu'à ce que ladite pression de remplissage du gaz dans ladite cuve (12, 112) soit atteinte ; mesurer une augmentation réelle de pression dans ladite cuve réservoir (12, 112) pour chacun desdits gaz composants introduits dans ladite cuve (12, 112) ; déterminer le rapport desdits gaz composants dans ladite cuve réservoir (12, 112) en déterminant le nombre de moles de chacun desdits gaz composants dans ladite cuve (12, 112) d'après ledit rapport initial connu des gaz composants dans ladite cuve ( 12, 112), ladite pression totale et lesdites variations de pression réelles mesurées pour chacun desdits gaz composants introduits dans ladite cuve (12, 112) ; dégager ledit gaz de ladite cuve (12, 112) à travers lesdits moyens de sortie (28, 128) ; mesurer une vitesse de variation de la pression du gaz dans la cuve (12, 112) au cours du temps, en présence d'un flux de sortie ; et commander (32, 132) ledit dégagement de gaz de ladite cuve (12, 112), caractérisé par :

la formation d'un signal différencié de pression représentant la vitesse de variation de pression dans ladite cuve réservoir (12, 112) pendant une certaine période de temps en présence d'un débit de sortie et lesdits moyens de commande du dégagement dudit gaz répondant audit signal différencié de pression basé sur la vitesse de variation de la pression du gaz détectée dans la cuve réservoir (12, 112) pendant une certaine période de temps.

8. Procédé selon la revendication 7, dans lequel ladite étape de commande (32, 132) dudit dégagement du gaz comprend : la comparaison (32, 132) de ladite variation de la pression du gaz avec une valeur de référence déterminée d'avance du flux de sortie, et la formation (32, 132) d'un signal d'erreur représentant la différence entre ladite variation de pression du gaz et ladite valeur du flux de sortie de référence ; et

l'ajustement (32, 132) dudit flux de sortie dans ladite sortie en réponse audit signal d'erreur pour réduire la différence entre ladite vitesse de variation de la pression du gaz et ladite valeur de référence du flux de sortie.

9. Procédé selon la revendication 7, comprenant encore les étapes suivantes :

comparer (32, 132) ladite pression mesurée du gaz dans ladite cuve avec une valeur de seuil déterminée d'avance ; et

introduire lesdits premier et second gaz composants dans ladite cuve (12, 112) avec ledit rapport souhaité, quand la pression dans ladite cuve (12, 112) descend au-dessous de ladite valeur de seuil et jusqu'à ce que la pression totale dudit gaz dans ladite cuve (12, 112) atteigne une limite supérieure déterminée d'avance.

10. Procédé selon la revendication 7, comprenant encore les étapes suivantes :

déterminer (32, 132) une pression définie d'avance dans ladite cuve (12, 112) après une inhalation suivante prédéterminée par le patient, d'après ladite vitesse détectée de variation de pression dans ladite cuve (12, 112) ;

comparer (32, 132) ladite pression prévue avec une valeur de seuil de pression déterminée d'avance ; et

remplir ladite cuve (12, 112) dudit gaz lorsque ladite pression prévue descend audessous de ladite valeur de pression de seuil déterminée d'avance.

11. Procédé selon la revendication 7, dans lequel ledit gaz respiratoire est dégagé de ladite cuve (12, 112) pendant un laps de temps déterminé d'avance en fonction d'une commande de flux de dégagement, et comprend encore les étapes suivantes :

a) retrancher (32, 132) ladite variation de pression mesurée en présence d'un flux de sortie d'une variation désirée de pression pour obtenir une erreur de variation de pression du gaz dans la quantité de gaz délivrée dans une sortie de gaz ;

b) intégrer (32, 132) ladite erreur de variation de pression du gaz pour chacune desdites délivrances afin de déterminer une erreur intégrée de la quantité de gaz délivrée ;

c) définir (32, 132) ladite commande de flux de dégagement pour le dégagement suivant à ladite variation souhaitée de pression plus une quantité proportionnelle à l'erreur intégrée ci-dessus ; et

d) répéter les étapes a) - c) pour une délivrance ultérieure d'un certain volume de gaz.

12. Procédé selon la revendication 7, comprenant encore les étapes suivantes :

mesurer (32, 132) le volume du flux dans lesdits moyens de sortie, basé sur les variations de la pression (30, 130) dudit gaz dans ladite cuve réservoir (12, 112) après inhalation par le patient ;

comparer (32, 132) ledit volume du flux dans lesdits moyens de sortie (28, 128) à un niveau de seuil de sortie inférieur déterminé d'avance et à un niveau supérieur de seuil de sortie déterminé d'avance ;

déclencher le flux de sortie (34, 35 ; 134, 135) dudit gaz à travers lesdits moyens de sortie (28, 128) quand ledit volume du flux à la sortie descend au-dessous dudit niveau inférieur de seuil ; et

arrêter le flux de sortie (34, 35 ; 134, 135) dudit gaz à travers lesdits moyens de sortie (28, 128) quand ledit volume du flux de sortie monte au-dessus dudit niveau supérieur de seuil déterminé d'avance.

FIG. 1

O₂ 17 16 OXYGEN INLET 20 24 12 14 30 10

CONTROL 32

AIR 19 18 AIR INLET 22 26 34 35 28

EP 0 482 261 B1

FIG. 2

EP 0 482 261 B1

EP 0 482 261 B1

Fig. 3

Fig. 4

20

## Fig. 5

## Fig. 6